# EUROPÄISCHE PATENTSCHRIFT

(11) **EP 1 335 684 B1**
(45) Veröffentlichungstag und Bekanntmachung des Hinweises auf die Patenterteilung: **12.01.2005**
(21) Anmeldenummer: 01997266.0
(22) Anmeldetag: 21.11.2001
(51) Int. Cl.: A61F 2/38

(54) **KNIEGELENK-ENDOPROTHESE-SYSTEM**
KNEE JOINT ENDOPROSTHESIS SYSTEM
ENDOPROTHESE POUR L'ARTICULATION DU GENOU

(30) Priorität: 21.11.2000 DE 10057675
(43) Veröffentlichungstag der Anmeldung: 20.08.2003
(73) Patentinhaber: Nowakowski, Andrej, 33154 Salzkotten (DE)
(72) Erfinder: Nowakowski, Andrej, 33154 Salzkotten (DE)
(74) Vertreter: Patentanwälte Thömen & Körner
(86) Internationale Anmeldenummer: PCT/DE2001/004329
(87) Internationale Veröffentlichungsnummer: WO 2002/041809

(56) Entgegenhaltungen:
- EP-A- 0 183 669
- WO-A-89/09579
- DE-A- 3 429 157
- DE-A- 19 816 984
- FR-A- 2 630 640
- US-A- 5 092 895

## Beschreibung

Die Erfindung betrifft ein Kniegelenk-Endoprothesensystem nach dem Oberbegriff des Anspruchs 1.

Kniegelenk-Endoprothesen dienen zur Wiederherstellung der Kniegelenkfunktion nach einer Schädigung des natürlichen Kniegelenks durch Verschleiß oder Knochenschädigung, z. B. durch einen Tumor.

Kniegelenk-Endoprothesensysteme sind bereits aus der DE 198 16 984 A1, der DE 197 08 375 A1, der DE 91 04 680, der FR 2 702 369 und der WO 89/09579 bekannt. Bei der gattungsbildenden WO 89/09579 sind zwei Transversalträger mit Abstand zu einer oberen Resektionsfläche der Tibia in die Tibia integriert. Zwei Gelenkflächeneinheiten sind über Gelenkflächenträger jeweils in einer Ausnehmung der Transversalträger abgestützt und mittels Madenschrauben fixiert. Die Ausnehmungen erstrecken sich axial des Transversalträgers und die Gelenkflächenträger sind nur von oben in die Ausnehmungen einsetzbar.

Um für die Montage der Gelenkflächeneinheiten mit den Gelenkflächenträger in den Transversalträgern einen ausreichen Freiraum zu erhalten, müssen der Oberschenkelknochen, also der Fermur und der Schienbeinknochen, also die Tibia gespreizt werden, was aber durch die Kreuzbänder begrenzt wird.

Daher ist bei den bekannten Endoprothesensystemen eine Resektion des vorderen Kreuzbandes erforderlich, obgleich die Bedeutung des vorderen Kreuzbandes als zentraler Stützpfeiler für die Kniestabilität als auch zur Unterstützung der physiologischen Kniebewegung bekannt ist.

Als Grund für die bekannten Maßnahmen sind Stabilitätsprobleme der bisher entwickelten Prothesen an sich, chirurgische

Probleme durch den Einsatz von proximal sowie die begrenzten Einstellmöglichkeiten der Prothesen in Ausrichtung zur Tibiaplateauneigung zu sehen.

Die der Erfindung zugrundeliegende Aufgabe besteht darin, ein Kniegelenk-Endoprothesensystem zu schaffen, dessen Tibiakomponente unter Erhalt der Kreuzbänder stabil in die Tibia einbaubar und fixierbar ist und so eine dauerhafte und physiologisch korrekte Beweglichkeit des Kniegelenks ermöglicht.

Diese Aufgabe wird bei einem Kniegelenk-Endoprothesensystem nach dem Oberbegriff des Anspruchs 1 durch dessen Merkmale gelöst.

Weiterbildungen und vorteilhafte Ausgestaltungen ergeben sich aus den Unteransprüchen.

Der Grundgedanke besteht darin, tibiaseitig zwei einzelne Gelenkflächeneinheiten auf einem eingebrachten Transversalträger auszurichten und zu fixieren. Hierdurch kann bei konstruktionsbedingter hoher Stabilität der Prothese der Erhalt der Kreuzbänder erreicht werden. Durch die Befestigung der Gelenkflächeneinheiten auf dem Transversalträger ist die achsengerechte Ausrichtung zueinander gewährleistet, wodurch ein vorzeitiger Verschleiß der Gleitlager vermieden wird.

Im Gegensatz zu bekannten Lösungen, bei denen der Transversalträger lediglich auf die oberen Resektionsfläche der Tibia aufgesetzt wird, ermöglicht die Integration in die Tibia mit Abstand zur Resektionsfläche eine besonders feste Verankerung.

Die Ausrichtung der Tibiaplateauneigung nach posterior, welche wesentlich zur Kreuzbandspannung und somit zur physiologischen Bewegung beiträgt, ist stufenlos nach Vorgabe des zu versorgenden Kniegelenks einstellbar.

Der Einbau der beiden Gelenkflächeneinheiten kann von vorn, oder zum kompletten Erhalt der ventralen Corticalis von schräg vorn oben, erfolgen. Zum herkömmlichen chirurgischen Verfahren ist eine kleine zusätzliche Incisur zum Einbringen des Transversalträgers erforderlich.

Vorzugsweise ist der Transversalträger etwa mittig in die Tibia integriert.

Da hierdurch die Achse des Transversalträgers näher an der Projektion des Schwerpunktes der Gelenkfläche liegt, wird eine Torsionsbelastung vermieden. Diese führte nämlich bei bekannten Lösungen mit dem Verbindungssteg häufig zu Materialermüdung und damit zum Bruch des Verbindungssteges.

Gemäß einer Weiterbildung weist der Transversalträger an wenigstens einem axialen Ende eine Schlüsselfläche oder eine Flansch auf.

Hierdurch kann der Transversalträger mittels eines Werkzeugs in die Tibia eingebracht und zur Einstellung des Winkels zur Ausrichtung der Tibiaplateauneigung justiert werden.

Vorzugsweise bestehen die Gelenkflächenträger jeweils aus einer Tibiaplateau-Platte und einer dazu senkrechten oder zur Senkrechten angewinkelten Stütze. Das untere Ende der Stütze ist in einer Ausnehmung des Transversalträgers befestigt.

Diese Ausgestaltung der Stütze minimiert die unvermeidliche Ausfräsung der Tibia und vermeidet so weitgehend eine Schwächung der Knochensubstanz. Die Hauptkraftkomponente verläuft in Richtung der Längsachse der Stütze, wodurch Torsions-, Scher- und Biegebelastungen reduziert werden. Eine Neigung der Stütze kommt dann in Betracht, wenn sich der Tibiakopf stark nach unten verjüngt.

In einer vorteilhaften Ausgestaltung bilden das untere Ende der Stütze und die Ausnehmung des Transversalträgers eine formschlüssige Verbindung.

Das untere Ende der Stütze kann bei der Montage quer zur Achse des Transversalträgers in diesen eingesetzt werden und ist in bezug auf die Hauptkraftkomponente anschließend dauerhaft fixiert.

Weiter ist vorgesehen, dass die Oberseite der Tibiaplateau-Platte mit ihrer zur oberen Resektionsfläche der Tibia zugewandten Unterseite einen spitzen Winkel einschließt.

Hierdurch ist eine Achsenkorrektur zur Achse des eingebrachten Transversalträgers möglich, um den erforderlichen Varus/Valguswinkel einzustellen.

Vorzugsweise ist die Außenfläche des Transversalträgers profiliert gestaltet und/oder weist Durchbrüche zu inneren Hohlräumen auf.

Hierdurch wird ermöglicht, dass Knochensubstanz in das Profil oder die Durchbrüche einwachsen kann, wodurch eine formschlüssige Verbindung zwischen der Knochensubstanz und dem Transversalträger während des Heilungsprozesses entsteht, was die sekundäre Stabilität erhöht.

Ferner kann die relative Lage zwischen den Gleitlagern und der Tibiaplateau-Platte durch eine Justiervorrichtung mit Führungen und Anschlägen veränderbar sein.

Durch diese Justiervorrichtung ist nach Einbau des Transversalträgers, der Stützen und der Tibiaplateau-Platte noch eine Feinjustierung der Gleitlager möglich, um eine physiologisch optimale Funktion des Kniegelenks zu gewährleisten.

Nachfolgend wird die Erfindung anhand eines Ausführungsbeispiels beschrieben, das in der Zeichnung dargestellt ist.

In der Zeichnung zeigen:
- Fig. 1 a, b: eine schematische Darstellung eines Kniegelenk-Endoprothesensystems,
- Fig. 2: eine Draufsicht auf das Transversalträger-Tibiaplateau und
- Fig. 3: eine Frontalansicht gemäß einer Ausführungsform nach der Erfindung mit einer weiteren Ausführungsform der Gelenkflächeneinheiten.

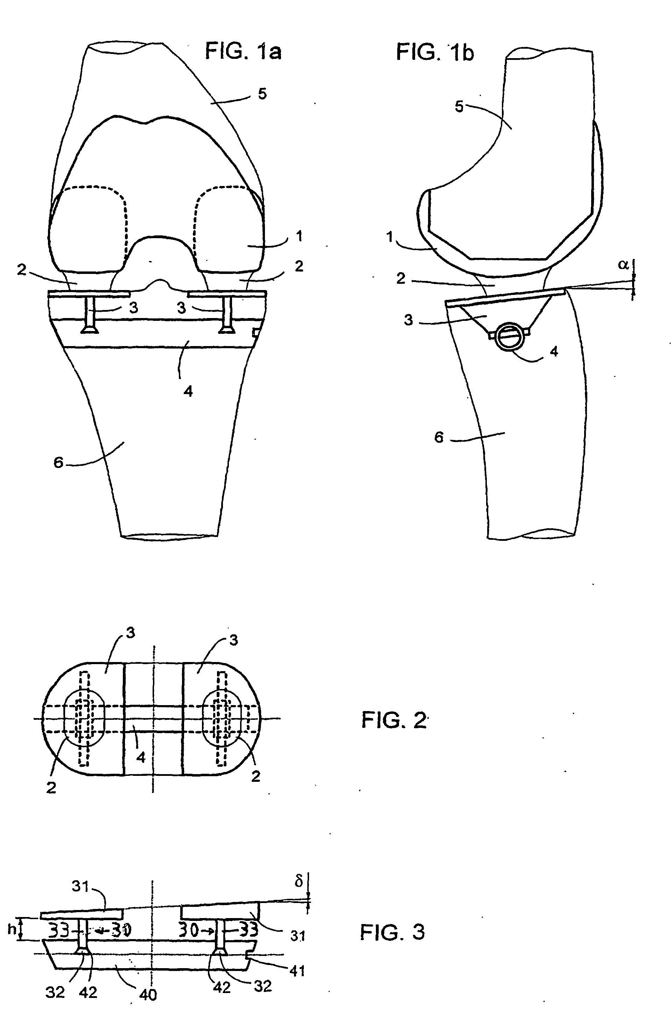

Die Figur 1a zeigt eine Femurkomponente 1 an einem Femur 5, welche über zwei Gleitlager 2 mit den Gelenkfächeneinheiten 3 des Transversalträger-Tibiaplateaus artikuliert. Die Gelenkflächeneinheiten 3 wiederum sind auf dem Transversalträger 4 fixiert. Das Tibiaplateau ist in eine Tibia 6 eingebracht. Bei der Femurkomponente handelt es sich um eine übliche Form, wobei es darauf ankommt, dass die Ausbildung der Kongruenz zum Gleitlager 2 im Winkelbereich des Gehens möglichst hochkonform ist.

Des weiteren sollte die Gleitfläche der Femurkomponente nach dorsal hin durch multiple Radien dargestellt werden, um die physiologischen Condylen möglichst gut nachzubilden, da hierdurch ebenfalls die Spannung der Kreuzbänder bestimmt wird. Die Ausbildung des Lagers zwischen den Gelenkflächeneinheiten 3 und den Gleitlagern 2 kann einfach plan erfolgen, oder zur Erhöhung der Stabilität durch Anschläge oder Führungen verändert werden.

Alle Auflageflächen der Prothesenkomponenten aus den zugelassenen Werkstoffen zum Knochen hin sind nach den gängigen Verfahren so ausgebildet, dass der Knochen einwachsen kann und somit die sekundäre Stabilität ausbildet. Zur Erhöhung der primären Stabilität kann der Transversalträger 4 mit einem Außengewinde versehen werden.

Die Figur 1b zeigt die Seitenansicht der Figur la. Wichtig ist hier, dass der röntgendiagnostisch präoperativ oder auf geeignete Weise intraoperativ festgestellte Winkel α zur Ausrichtung der Tibiaplateauneigung nach dorsal genau nachgebildet wird, um die physiologischen Kreuzbandspannungen während des Bewegungsablaufs beizubehalten.

Die in der Figur 2 dargestellte tibiaseitige Gleitfläche der Gleitlager 2 kann aufgrund der vollen Auflagefläche kleiner als die femurseitige Gleitfläche ausgebildet werden. Im Gegensatz zur femurseitigen Gleitfläche, welche bei Beugung durch den kleiner werdenden Condylenradius abnimmt, ist hier somit nicht mit stärkerem Verschleiß der aus vorzugsweise UHMW-Polyethylen gefertigten Gleitlager 2 zu rechnen.

Bei Ausführungsformen mit Anschlägen oder Führungen muss die tibiaseitige Gleitfläche der Gleitlager 2 gegebenenfalls vergrößert werden.

In der Figur 3 sind noch einmal die wesentlichen Bestandteile des Transversalträger-Tibiaplateaus mit 2 Gelenkflächeneinheiten 3, bestehend aus 30 bis 32, und einem Transversalträger 4, bestehend aus 40 bis 42, aufgezeigt.

Die durch geeignete Längenwahl der Gelenkflächenträger 30 festgelegte Höhe h bestimmt den festen Sitz und führt somit ebenfalls zu hoher Primärstabilität.

Sollte intraoperativ eine zusätzliche Achsenkorrektur zum eingebrachten Transversalträger 4 erforderlich sein, kann diese durch eine weitere Ausführungsform der Gelenkflächeneinheiten durch Änderung des Varus-/Valguswinkels δ erfolgen. Die Verbindung zwischen den Gelenkflächeneinheiten 3 und dem Transversalträger 4 ist als Ausführungsform mit Schwalbenpaßnut 32,42 dargestellt. Andere Verbindungsmöglichkeiten nach dem Stand der Technik sind hier ebenfalls anwendbar.

Die Grundform des Transversalträgers 40, in Figur 3 als Ausführungsform mit rundem Hohlprofil dargestellt, kann auch als Schraube, Hohlschraube oder durch andere Profile oder Hohlprofile ausgebildet werden, welche Durchbrechungen zum Knocheneinwuchs aufweisen können. Die Länge sollte so gewählt werden, dass er beidseitig in der Corticalis der Tibia verankert ist.
Bei sich stark verjüngendem Tibiakopf kann es erforderlich werden, die Ausrichtung zwischen Gelenkflächenträger 30 und Gelenkfläche 31 nicht senkrecht zueinander zu gestalten. Der Transversalträgerflansch 41 dient zum Einbringen des Transversalträgers 4 in die Tibia, sowie zur Einstellung des Winkels α zur Ausrichtung der Tibiaplateauneigung nach dorsal. Durch geeignete Form kann hieran das Resektionswerkzeug zur Tibiagelenkflächenresektion ausgerichtet und befestigt werden.

## Patentansprüche

1. Kniegelenk-Endoprothesensystem mit einer Femurkomponente (1) , zwei Gleitlagern (2) sowie einer Tibiakomponente, wobei die Tibiakomponente zwei Gelenkflächeneinheiten (3) und einen Transversalträger (4) umfasst, der Transversalträger (4) mit Abstand zu einer oberen Resektionsfläche der Tibia (6) in die Tibia (6) zu integrieren ist und die Gelenkflächeneinheiten (3) über Gelenkflächenträger (30) jeweils in einer Ausnehmung (42) des Transversalträger (4) abgestützt und fixiert sind, **dadurch gekennzeichnet, dass** die Ausnehmungen (42) quer zur Achse des Transversalträgers (4) verlaufen und zum Einsetzen der Gelenkflächenträger (30) bezogen auf das Kniegelenk von vorn, also ventral zugänglich sind.

2. Kniegelenk-Endoprothesensystem nach Anspruch 1, **dadurch gekennzeichnet, dass** der Transversalträger (4) etwa mittig in die Tibia (6) zu integrieren ist.

3. Kniegelenk-Endoprothesensystem nach Anspruch 1 oder 2, **dadurch gekennzeichnet, dass** der Transversalträger (4) an wenigstens einem axialen Ende eine Schlüsselfläche oder einen Flansch (41) aufweist.

4. Kniegelenk-Endoprothesensystem nach einem der Ansprüche 1 bis 3 , **dadurch gekennzeichnet, dass** die Gelenkflächenträger (30) jeweils aus einer Tibiaplateau-Platte (31) und einer dazu senkrechten oder zur Senkrechten angewinkelten Stütze (33) bestehen und das untere Ende (32) der Stütze (33) in einer Ausnehmung (42) des Transversalträgers (4) befestigt ist.

5. Kniegelenk-Endoprothesensystem nach einem der Ansprüche 1 bis 4, **dadurch gekennzeichnet, dass** das untere Ende (32) der Stütze (33) und die Ausnehmung (42) des Transversalträgers (4) eine formschlüssige Verbindung bilden.

6. Kniegelenk-Endoprothesensystem nach Anspruch 4 oder 5, **dadurch gekennzeichnet, dass** die Oberseite der Tibiaplateau-Platte (31) mit ihrer zur oberen Resektionsfläche der Tibia (6) zugewandten Unterseite einen spitzen Winkel (δ) einschließt.

7. Kniegelenk-Endoprothesensystem nach einem der Ansprüche 1 bis 6, **dadurch gekennzeichnet, dass** die Außenfläche des . Transversalträgers (4) profiliert gestaltet ist und/oder Durchbrüche zu inneren Hohlräumen aufweist.

8. Kniegelenk-Endoprothesensystem nach einem der Ansprüche 4 bis 7, **dadurch gekennzeichnet, dass** die relative Lage zwischen den Gleitlagern (2) und der Tibiaplateau-Platte (31) durch eine Justiervorrichtung mit Führungen und Anschlägen veränderbar ist.

## Claims

1. A knee joint endoprosthesis system having a femur component (1), two friction bearings (2) and a tibia component, whereby the tibia component comprises two joint surface units (3) and a transversal support (4), the transversal support (4) is to be integrated into the tibia (6) at a distance from an upper resection surface of the tibia (6), and the joint surface units (3) being supported on and attached to a recess (42) in the transversal support (4) via the joint surface support (30), **characterized in that** the recesses (42) run across the axis of the transversal support (4) and are accessible from the front, i.e., ventrally, for insertion of the joint surface supports (30).

2. The knee joint endoprosthesis system according to Claim 1, **characterized in that** the transversal support (4) is to be integrated into the tibia (6) approximately at the center.

3. The knee joint endoprosthesis system according to Claim 1 or 2, **characterized in that** the transversal support (4) has a key surface or a flange (41) on at least one axial end.

4. The knee joint endoprosthesis system according to one of Claims 1 through 3, **characterized in that** the joint surface supports (30) each consist of a tibia plateau plate (31) and a support element (33) perpendicular to it or bent to the perpendicular, and the lower end (32) of the support element (33) is secured in a recess (42) in the transversal support (4).

5. The knee joint endoprosthesis system according to one of Claims 1 through 4, **characterized in that** the lower end (32) of the support element (33) and the recess (42) of the transversal support (4) create a form-fitting connection.

6. The knee joint endoprosthesis system according to this invention Claim 4 or 5, **characterized in that** the upper side of the tibia plateau plate (31) forms an acute angle (δ) with its bottom side which faces the upper resection surface of the tibia (6).

7. Knee joint endoprosthesis system according to one of Claims 1 through 6, **characterized in that** the outside surface of the transversal support (4) is designed with profiling and/or has openings toward interior cavities.

8. The knee joint endoprosthesis system according to one of Claims 4 through 7, **characterized in that** the relative position between the friction bearings (2) and the tibia plateau plate (31) is variable by means of an adjusting device having guides and stops.

## Revendications

1. Système d'endoprothèse de l'articulation du genou avec une composante fémorale (1), deux paliers glissants (2) ainsi qu'une composante tibiale, à l'occasion de quoi la composante tibiale englobe deux unités de surface d'articulation (3) et un support transversal (4), le support transversal (4) devant être inséré dans le tibia (6) avec un intervalle à une surface de résection supérieure du tibia (6), et les unités de surface d'articulation (3) étant logées et fixées respectivement, via des supports de surface d'articulation (30), dans un logement (42) du support transversal (4), **caractérisé en ce que** les logements (42) sont disposés transversalement à l'axe du support transversal (4) et sont accessibles par l'avant, donc de façon ventrale, par rapport à l'articulation du genou pour la mise en place des supports de surface d'articulation (30).

2. Système d'endoprothèse de l'articulation du genou selon la revendication 1, **caractérisé en ce que** le support transversal (4) doit être intégré à peu près de façon centrée dans le tibia (6).

3. Système d'endoprothèse de l'articulation du genou selon la revendication 1 ou 2, **caractérisé en ce que** le support transversal (4) présente, au moins à une extrémité axiale, une surface de clé ou une bride (41).

4. Système d'endoprothèse de l'articulation du genou selon l'une des revendications 1 à 3, **caractérisé en ce que** les supports de surface d'articulation (30) se composent chacun d'une plaque de plateau de tibia (31) et d'un appui (33) vertical faisant un angle par rapport à cette plaque ou à la verticale et **en ce que** l'extrémité inférieure (32) de l'appui (33) est fixée dans un logement (42) du support transversal (4).

5. Système d'endoprothèse de l'articulation du genou selon l'une des revendications 1 à 4, **caractérisé en ce que** l'extrémité inférieure (32) de l'appui (33) et le logement (42) du support transversal (4) forment une liaison mécanique.

6. Système d'endoprothèse de l'articulation du genou selon la revendication 4 ou 5, **caractérisé en ce que** le côté supérieur de la plaque de plateau de tibia (31) inclut un angle aigu (δ) avec son côté inférieur tourné vers la surface de résection supérieure du tibia (6).

7. Système d'endoprothèse de l'articulation du genou selon l'une des revendications 1 à 6, **caractérisé en ce que** la surface extérieure du support transversal (4) est constituée de façon profilée et/ou présente des traversées vers des cavités internes.

8. Système d'endoprothèse de l'articulation du genou selon l'une des revendications 4 à 7, **caractérisé en ce que** la position relative entre les paliers glissants (2) et la plaque de plateau de tibia (31) peut être modifiée par un dispositif d'ajustement avec des guidages et des butées.
